# EUROPEAN PATENT SPECIFICATION

(11) **EP 1 796 700 B1**
(45) Date of publication and mention of the grant of the patent: **25.04.2012**
(21) Application number: 04770696.5
(22) Date of filing: 23.08.2004
(51) Int. Cl.: A61K 36/06

(54) **EUKARYOTIC BASED SYNERGISTIC FORMULATION FOR GASTRO-INTESTINAL DISORDERS**
SYNERGISTISCHE FORMULIERUNG AUF EUKARYOTISCHER BASIS GEGEN GASTROINTESTINALE ERKRANKUNGEN
FORMULATION SYNERGISTIQUE EUCARYOTE POUR TROUBLES GASTRO-INTESTINAUX

(43) Date of publication of application: 20.06.2007
(73) Proprietor: Bharat Biotech International Limited, Hyderabad 500 078 (IN)
(72) Inventor: ELLA, Krishna, Murthy, Hyderabad 500 078 (IN); VELLIMEDU, Srinivas, Kannappa, Hyderabad 500 078 (IN); SUNKARA, Sivasankara, Rao, Hyderabad 500 078 (IN)
(74) Representative: Meyer-Dulheuer, Karl-Hermann
(86) International application number: PCT/IN2004/000256
(87) International publication number: WO 2006/021965

(56) References cited:
- EP-A1- 1 481 682
- GB-A- 930 107
- US-A- 4 595 590
- US-A- 5 501 857
- DATABASE MEDLINE [Online] US NATIONAL LIBRARY OF MEDICINE (NLM), BETHESDA, MD, US; 1 August 2004 (2004-08-01), ERDEVE OMER ET AL: "The probiotic effect of Saccharomyces boulardii in a pediatric age group." XP002325265 Database accession no. NLM15357564 & JOURNAL OF TROPICAL PEDIATRICS. AUG 2004, vol. 50, no. 4, 1 August 2004 (2004-08-01), pages 234-236, ISSN: 0142-6338
- DATABASE BIOSIS [Online] BIOSCIENCES INFORMATION SERVICE, PHILADELPHIA, PA, US; 1994, MCFARLAND LYNNE V ET AL: "A randomized placebo-controlled trial of Saccharomyces boulardii in combination with standard antibiotics for Clostridium difficile disease" XP002325266 Database accession no. PREV199497356313 & JAMA (JOURNAL OF THE AMERICAN MEDICAL ASSOCIATION), vol. 271, no. 24, 1994, pages 1913-1918, ISSN: 0098-7484
- DATABASE BIOSIS [Online] BIOSCIENCES INFORMATION SERVICE, PHILADELPHIA, PA, US; 1993, KLEIN SUSAN M ET AL: "Recovery and elimination of the biotherapeutic agent, Saccharomyces boulardii, in healthy human volunteers" XP002325267 Database accession no. PREV199497046990 & PHARMACEUTICAL RESEARCH (NEW YORK), vol. 10, no. 11, 1993, pages 1615-1619, ISSN: 0724-8741
- GIROLA M ET AL: "Efficacy of probiotic preparation with living, freeze-dried lactic acid bacteria and yeast on child diarrhoea" BIOSIS, ARCHIVO DI MEDICINA INTERNA, vol. 47, no. 2-3, 1995, pages 61-72, XP002035135
- DATABASE BIOSIS [Online] BIOSCIENCES INFORMATION SERVICE, PHILADELPHIA, PA, US; 1989, GARDNER E T ET AL: "STUDIES ON THE MICROBIOLOGY AND PRODUCTION OF PINEAPPLE ANANAS-COMOSUS WINE" XP002325268 Database accession no. PREV199089013877 & MICROBIOS, vol. 59, no. 239, 1989, pages 85-92, ISSN: 0026-2633
- CREMONINI F ET AL: "Probiotics in antibiotic-associated diarrhoea", DIGESTIVE AND LIVER DISEASE, vol. 34, no. Supplement 2, September 2002 (2002-09), pages S78-S80, ISSN: 1590-8658
- SAAVEDRA JOSE: "Probiotics and infectious diarrhea", AMERICAN JOURNAL OF GASTROENTEROLOGY, vol. 95, no. 1 Suppl., January 2000 (2000-01), pages S16-S18, ISSN: 0002-9270
- GAON DAVID ET AL: "Effect of Lactobacillus strains and Saccharomyces boulardii on persistent diarrhea in children.", MEDICINA (BUENOS AIRES), vol. 63, no. 4, 2003, pages 293-298, XP008131317, ISSN: 0025-7680
- GIRARD-PIPAU F ET AL: "Intestinal microflora, short chain and cellular fatty acids, influence of a probiotic Saccharomyces boulardii.", MICROBIAL ECOLOGY IN HEALTH AND DISEASE, vol. 14, no. 4, November 2002 (2002-11), pages 220-227, XP008131318, ISSN: 0891-060X

## Description

### FIELD OF INVENTION

The invention provides a method for producing a formulation that regulates the balance of microbial flora in the gastrointestinal tract of mammals. More particularly, the present invention provides a method for producing a eukaryotic based probiotic formulation with more stability. Still more particularly, the method of the present invention provides a formulation, which reduces if not prevents the ill effects of the harmful organisms.

Yet more particularly, the formulation obtained by method of the present invention stimulates the growth of probiotics and inhibits the growth of undesired harmful microbes. Further, the formulation obtained by method also takes care of secondary ill effects induced through ingestion or administration of pharmaceuticals or physiological components, to which the gut-micro-flora is associated. The formulation obtained by method proved to be useful as preventive and curative therapy for gastrointestinal disorders arising through bacterial and or viral infection, particularly when used in combination with prokaryotic organisms.

### BACKGROUND OF THE INVENTION:

It is a known fact that the human body consists of about 10 trillion cells, but it is the home to 10 times as many bacterial cells as human cells. Indeed, about 100 trillion bacterial cells, from more than 500 different species, reside in the human gut, lungs, and skin. Some of these organisms can cause disease while most are beneficial. These "good microbes" help defend the body against disease-causing microbes by a variety of physiological mechanisms.

The gastrointestinal tract (GI tract) is a complex micro-ecosystem, in which the mucosal lining hosts billions of microorganisms that live attached to it. Imbalance of this ecosystem results in gastrointestinal disorders. Probiotics or life promoting, are a class of microorganisms defined as live microbial organisms that beneficially affect the animal and human hosts. Some of the probiotics are of transient nature and are introduced through food or food supplement. The probiotics, inhabitant of the mucosal membrane, reproduce in the digestive tract thereby improving the microbial balance of the intestinal microflora or improving the properties of the indigenous microflora. The beneficial effects of probiotics may be mediated by a direct antagonistic effect against specific groups of organisms resulting in a decrease in numbers, by an effect on their metabolism or by stimulation of immunity. Probiotics also suppress viable counts of an undesired organism by producing antibacterial compounds, by competing for nutrients or for adhesion sites. Further, they may alter microbial metabolism by increasing or decreasing enzyme activity or they may stimulate the immune system by increasing antibody levels or increasing macrophage activity.

The beneficial micro organisms can produce substances, such as lactic acid, hydrogen peroxide, and potent antibiotic compounds called bacteriocins, that inhibit the growth of harmful bacteria and fungi. They also compete with disease-causing microbes for nutrients and space, thus hindering overgrowth of the bad bugs. Beneficial organisms also secrete enzymes that aid digestion and produce substances that raise intestinal acidity, thereby promoting the absorption of calcium, magnesium, and zinc.

These probiotics boost immune function, reduce food allergies, and prevent certain infections. Preliminary studies suggest certain good bacteria may also have cholesterol-lowering and anticancer properties. Probiotics also appear to stem from their effects on the lymphoid tissue in the gut.

Regular ingestion of probiotic bacteria actually modifies both immune-enhancing and immune-suppressing cells in the gut, which helps modulate the immune response. Thus, probiotics can heighten the body's response to viruses, while decreasing the response to certain antigens, such as those responsible for food allergies." Proper balancing of probiotics is very important. According to researchers in Europe the imbalance of this micro-flora is responsible for as much as 80% of degenerative diseases. Without understandding the actual role played by probiotics, the lactic acid producing probiotics are in use for processing food and beverages over 4000 years. Traditionally, the balance is partly maintained by ingesting food containing these organisms. A considerable amount of research has been conducted on probiotics in past decades. The benefits of probiotic are well documented but are not evidenced adequately. It is well known that administration of certain drugs including antibiotics results in reducing the micro-flora (prokaryotic) inhabitant to the gastrointestinal tract & beneficial to the mammals but are sensitive to such antibiotics. Hence, to counteract this resulting imbalance, in general, vitamins are administered simultaneously, which in turn helps in rebuilding the desired level of micro-flora.

Relevant prior art known to the inventor includes US Patent No. 4,806,368. This patent claims a dietary fibre based vitamin, mineral and beneficial bacteria tablet with enhanced bacterial viability. The tablets are used as nutritional supplement. In accordance with this patent the tablet essentially comprises of (i) apple fibre including insoluble fibre element, (ii) Lyophilised live bacteria, (iii) vitamin having antioxidant property, (iv) an amino acid having reducing property, (v) an alkaline mineral salt and means of stimulating bacterial growth. The live bacteria used are lactic acid producing bacteria and stimulant used are selected from autolysed yeast extract, acid whey and enzyme hydrolysed casein. Further, *Lactobacillus acidophilus* either as tablets or powder sold in health food stores and comprising *L. acidophilus,* carboxymethyl cellulose etc. have short term potency because of the rapid inactivation of *L. acidophilus* in the human stomach where the pH is around 2 to 3. The problem has been solved by introducing apple fibre supplemented with vitamins etc. The fibre mainly used as dietary supplement, on reconstitution, has exceptionally protective and stimulatory effect on growth of probiotics.

US Patent No. 5,501,857 discloses again a nutritional and dietary composition, which combines certain incompatible substances separated from each other via multiple encapsulations. The microorganisms used are a combination of yeast, fungi and gastrointestinal bacteria. The method becomes cost extensive and requires special infrastructure for capsule-in-a- capsule structure.

US 6,468,525 relates to a food supplement formulation comprising 5 specific microflora (bacteria) in addition to other ingredients such as I glutamine, glycosamine, in order to support replacing mucosal linings particularly gastro-intestinal lining.

US 6,426,099 discloses herbal formulation for reestablishing intestinal bacteria.

US 2003/0104091 teaches a novel probiotic, prokaryotic-based formulation and a method for reducing pathogenic bacteria. Particularly the composition comprises a genus Bacillus.

US 5,773,000 advocates treatment of *Clostridium difficile* associated diseases such as colitis and antibiotic associated diarrhea by administering specific antibodies in combination with vancomycin, bacitracin, or metronidazole etc. However this treatment is not effective as relapses occur in about 10 to 20% of the cases.

The primary etiology of antibiotic-associated diarrhea has been *Clostridium difficile.* The indigenous microflora of a healthy individual suppresses the normally present *C. difficile.* However, when the indigenous microflora are disrupted (e.g., during antibiotic treatment) overgrowth of *C. difficile* may occur causing diarrhea and colitis. Though, treatment of *C. difficile* with antibiotics has proven effective, many times relapse occurs.

Generally, these diseases develop as a result of the production of two large toxins, toxin A (M.sub.r, 308,000) and toxin B (M.sub.r, 279,000), by the organism in the colon. Toxin A is believed to cause most of the gastrointestinal symptoms because of its enterotoxic activity in experimental animals. There is some evidence suggesting that the toxins act synergistically during the course of the disease and that the initial tissue damage caused by toxin A allows toxin B to exert its toxic effect.

Immunoprophylaxis has also been suggested as a type of treatment. It is known that vaccination against toxins A and B stimulates active immunity against *C. difficile* disease in experimental animals. At the present time, however, suitable vaccines against the organism and its toxins have not been developed for individuals at high risk, and it is still unclear whether active immunization is appropriate.

Alternatively, treatment by passive immunization has been suggested. In preliminary studies, serum antibodies against a toxigenic isolate of *C. difficile* protected hamsters against *C. difficile* disease when administered orally to the animals. Thus, passive immunity may be beneficial for prophylactic treatment.

Passive immunization with bovine antibodies has been examined as a possible alternative therapy in the treatment of other infectious diseases of the gastrointestinal tract, including diseases caused by rotavirus, enteropathogenic and enterotoxigenic *Escherichia coli, Vibrio cholerae,* and *Cryptosporidium parvum,* and the results indicate that antibodies administered in this manner provide protection.

Passive immunity from bovine antibodies offers the advantages that most animals and humans tolerate the material given orally and that the predominant antibody species present, immunoglobulin G1 (IgG1), is relatively resistant to proteolysis.

US Patent 6,010,695 describes therapeutically effective amount of an indigestible oligosaccharides inhibits the infection of mammals by *C. difficile* when administered enterally. Probably, the oligosaccharides stimulate the growth of probiotics there by suppressing *C. difficile* (competition for survival) and inturn reducing its ill effect.

Two controlled trials have found that the probiotic *E. coli* achieved results comparable to those of mesalamine, a standard drug used to treat ulcerative colitis.

In addition, a multicenter trial recently reported in Gastroenterology showed that probiotics of chronic colitis inflammatory condition often afflicts patients with ulcerative colitis who have undergone surgical resection of the colon. Probiotics' possible role in managing Crohn's disease is still unclear.

Similar to gastrointestinal disorders, fatty liver is a disorder commonly associated with obesity and diabetes, and recent findings suggest that increased production of ethanol due to the overgrowth of certain intestinal bacteria in obese individuals may contribute to the development of this disorder. This endorses the need to maintain the balanced ecosystem to mitigate and or avoid gastrointestinal disorder and its consequences.

In animal studies it has been seen that probiotic therapy for one month reduced chronic inflammation and fatty infiltration of the liver in obese mice with fatty liver. This finding supports the idea that overgrowth of ethanol-producing bacteria in the intestine may well play a role in fatty liver. A human trial is planned in order to gather proper evidence for the proposed therapy.

Numbers of studies have shown that the probiotic therapy may be helpful for disorders unrelated to the gastrointestinal system. For example, there is evidence that probiotics can reduce the rate of respiratory infections in children, enhance immunity in the elderly, protect against cavities, and combat bacterial vaginosis, a common infection of the reproductive tract in women. It may also improve lactose intolerance and reduce food-related allergies.

Patent 6,306,391 describes the drawbacks of commercially available combinations of lactobacillus and anti-infective agents into one formulation. It is reported that it is a mere admixture. Further, their study reports the non-viability of the organism beyond 7 days and poor functioning of the formulation in general.

In order to solve this problem, they devised a stable fixed dose oral pharmaceutical formulation of at least one anti-infective agent and at least one microorganism, wherein the agent and/or the microorganism is first coated to provide protective barrier around it. Next, the process involves a step of combining the agent and the microorganism susceptible to the anti-infective agent used into a single pharmaceutical formulation in the form of a capsule or a tablet.

The barrier protects the microorganism from the effect of the anti-infective agent to maintain the microorganism in a viable form for a period of at least three months. The agent can be an antibiotic such as amoxycillin and the microorganism can be *Lactobacillus acidophilus.*

Their studies have shown the stability of the capsules for a period of three to thirty six months. However, the protective barrier is essential in order to prevent direct contact of the organism with anti-infective agent to reduce its ill effect on microorganism.

*S.boulardii* is a benefical probiotic, non-colonizing yeast species. It supports the function of GI tract where it can aggressively displace problematic yeast species, exemplified by candida, increasing levels of IgA, inactivate bacterial toxins and release benefical polymers support establishment of friendly bacteria and help restore nutrient absorption capacity to the small intestine mucosa. It is resistant to gastric acidity and proteolysis and when taken orally becomes quickly established blooming soon after supplementation has began. In the gut it produces some B vitamins and as it becomes established it eliminates unfriendly strains of yeast such as *S. candida.* During use, friendly probiotic bacteria are able to colonize in the GI tract supporting micro ecology.

*S. boulardii* has been used in Europe for many decades to treat diarrhea. Clinical trials have shown it effective in preventing or treating diarrhea brought on by antibiotics. Further studies have shown it can support gut function with many conditions, including food allergies, parasites, Crohn's disease, Candida, Salmonella, travelers' diarrhea, HIV diarrhea and Pseudomonas. Clinical studies also demonstrate the protective effect of *S. boulardii* in intestinal infections, including *Clostridium difficile* and cholera. The protective effect of *S. boulardii* involves several types of activity in the epithelial tissue of the digestive tract, including inactivation of bacterial toxins, stimulation of intestinal immune response, and release of polyamines. Further, these preparations are not commercially available, which indicates that the therapy is not yet well established and proven. Moreover, to prevent ill effects of anti-infective agents, one has to administer *S. boulardii* separately there by requiring administration of two components making the process cumbersome and decreasing compliance to a patient. Additionally, coordinating administration and action of these two different components may pose problems.

It may be noted that most of the existing formulations are prepared using prokaryotics, preferably lactic acid producing bacteria with adjunct in the form of antibodies, antibiotics, drugs, other probiotics that are prokaryotics in origin, plant or herbal origin fibres and stimulants. Further, these formulations are generally used as nutrient or food supplement or dietary formulations. The formulations used to reducing the pathogenic organisms, prokaryotics are either administered separately in coordination with antibiotics. Prokaryotics, when used in combination with anti-infective agent such as antibiotics, are required to be coated with protected barrier to prevent its direct contact with the anti-infective agent. The direct contact has to be avoided in order to take care of the inactivation or reduction in potency and or efficiency of the organism arising through such contacts. In turn the fatal effects arising through such contact are also taken care of. There are no reports on the single formulation consisting of eukaryotics preferably *S.boulardii* and adjuncts such as other probiotics, other physiologically and pharmaceutically acceptable and compatible components including anti-infective agents in one formulation while maintaining efficiency.

Further relevant prior art may be found in the following documents D1-D12:
- D1:: GB 930 107 A (GIUSEPPE CARLO SIGURTA) 3 July 1963 (1963-07-03)
- D2:: US NATIONAL LIBRARY OF MEDICINE (NLM), BETHESDA, MD, US; 1 August 2004 (2004-08-01),
ERDEVE OMER ET AL: "The probiotic effect of Saccharomyces boulardii in a pediatric age group.", Database accession no. NLM15357564; & JOURNAL OF TROPICAL PEDIATRICS. AUG 2004, vol. 50, no. 4, 1 August 2004 (2004-08-01), pages 234-236, ISSN : 0142-6338
- D3:: BIOSCIENCES INFORMATION SERVICE, PHILADELPHIA, PA, US; 1994,
MCFARLAND LYNN E V ET AL: "A randomized placebo-controlled trial of Saccharomyces boulardii in combination with standard antibiotics for Clostridium difficile disease", Database accession no. PREV199497356313 ; & JAMA (JOURNAL OF THE AMERICAN MEDICAL ASSOCIATION), vol. 271, no. 24,1994, pages 1913-1918,ISSN: 0098-7484
- D4:: BIOSCIENCES INFORMATION SERVICE, PHILADELPHIA, PA, US; 1993,
KLEIN SUSAN M ET AL: "Recovery and elimination of the biotherapeutic agent, Saccharomyces boulardii, in healthy human volunteers", Database accession no. PREV199497046990 ; & PHARMACEUTICAL RESEARCH (I\IEW YORK), vol. 10, no. 11, 1993, pages 1615-1619, ISSN: 0724-8741
- D5:: GIROLA M ET AL: "Efficacy of probiotic preparation with living, freeze-dried lactic acid bacteria and yeast on child diarrhoea", BIOSIS, ARCHIVO 01 MEDICINA INTERNA, vol. 47, no. 2-3,1995, pages 61-72, XP002035135,
- D6:: US 4595590 A (HUBLOT ET AL) 17 June 1986 (1986-06-17)
- D7:: BIOSCIENCES INFORMATION SERVICE, PHILADELPHIA, PA, US; 1989,
GARDNER E T ET AL: "STUDIES ON THE MICROBIOLOGY AND PRODUCTION OF PINEAPPLE ANANAS-COMOSUS WINE", Database accession no. PREV199089013877 ; & MICROBIOS, vol. 59, no. 239, 1989, pages 85-92, ISSN: 0026-2633
- D8:: EP 1 481 682 A 1 (NUTRICIA NV [NL]) 1 December 2004 (2004-12-01)
- D9:: CREMONINI F ET AL: "Probiotics in antibiotic-associated diarrhoea", DIGESTIVE AND LIVER DISEASE, vol. 34, no. Supplement 2, September 2002 (2002-09), pages S78-S80, ISSN: 1590-8658
- D10:: SAAVEDRA JOSE: "Probiotics and infectious diarrhea", AMERICAN JOURNAL OF GASTROENTEROLOGY, vol. 95, no. 1 Suppl., January 2009 (2000-01), pages S16-S18, ISSN: 0002-9270
- D11:: GAON DAVID ET AL: "Effect of Lactobacillus strains and Saccharomyces boulardii on persistent diarrhea in children:", MEDICINA (BUENOS AIRES), vol. 63, no. 4, 2003, pages 293-298, ISSN: 0025-7680
- D12:: GIRARD-PIPAU F ET AL: "Intestinal microflora, short chain and cellular fatty acids, influence of a probiotic Saccharomyces boulardiL", MICROBIAL ECOLOGY IN HEALTH AND DISEASE, vol. 14, no. 4, November 2002 (2002-11), pages 220-227, ISSN: 0891-060X

D 1 discloses a formulation consisting of a solid stable suspension of a yeast culture *(S. cerevisiae, S. fragilis, S. delbrueckii, S. lactis, S. chevalieri) "in an anhydrous sugar-like medium obtained by heating to* 135 to 145°C *glucose. sucrose or corn-syrup, or any mixture thereof'.* It does not teach or suggest a synergistic formulation comprising S. *boulardii* or a method for producing the formulation comprising the various steps.

D5 discloses a formulation comprising *S cerevisiae* and vitamins. However, it does not teach or suggest a synergistic formulation comprising *S. boulardii* or a method for producing the formulation comprising the various steps listed in new claim 1.

D2 to D4, D6 and D9 to D 12 disclose the use of S. *boulardii* in the treatment of gastrointestinal disorders, however, none of these documents teaches or suggests a single synergistic formulation comprising *S. boulardii* and a further pharmaceutically or physiologically acceptable component, and do not disclose a method for producing such formulation.

D7 discloses a list of various bacteria and fungi that can be isolated from the skin pulp and fermenting must of pineapple (Ananas comosus), but do not suggest that *S. boulardii* can be isolated from Ananas comosus.

D8 relates to a preparation that contains oligosaccharides and probiotics preferably chosen from *Lactobacillus* or *Bifidobacterium* species or from *Saccharomyces* species.

We have discovered that the eukaryotics with a defined nucleus have a better survival rate over prokaryotics under diverse environmental conditions. Further, the eukaryotics when mixed with other anti-infective agents are not affected adversely like prokaryotics thereby increasing the potency of the formulation. Thus the formulation comprising of eukaryotics and pharmaceutically acceptable substances show improved efficiency and shelf life. Eukaryotics, particularly yeast can be stored while maintaining its potency for a long period and the potency can be revived only at a targeted site. Additionally, yeast stimulates the growth of probiotics inhabitant to gastrointestinal tract and maintains the balance of the ecosystem, which in turn prevents and or minimizes the dominance of pathogenic organisms and ill effects of the products produced by such harmful micro-flora. Eukaryotics, specifically Yeast through its metabolites enhances effective assimilation of minerals and other nutritional components. Amongst eukaryotics yeast is preferable and among various species of yeast *S. boulardii* is more preferable in view of having better growth rate as compared to other species such as *S cerevisiae,* which has been reported for being used as dietary adjuncts.

The novelty of the invention resides in using a eukaryotics particularly yeast, more particularly the specific yeast grown in a specific manner in combination with physiologically and or pharmaceutically acceptable components in one formulation, without using any protective barriers, while maintaining its efficiency and potency. While using single formulation, the invention becomes cost effective by reducing the cost on providing barriers for isolation of the components used. It also makes proper coordinated action of the components and reduces the administration of two drugs proving user friendly. Additionally, the yeast used is in such a state that it gets activated only on reaching the desired site i.e. gastrointestinal tract. Moreover, yeast does not react with adjunct during its storage and travel to the gastrointestinal tract and also does not get inactivated. Being biologically evolved the organism stands better chances of survival through mere competition. It acts in synergism either with anti-infective agent or food supplements to maintain balance of ecosystem through various modes as herein before described. This in turn combats the diseases arising through dominance of undesired organisms and its metabolic products such as toxins.

The main object of the present invention is to provide a method for producing a eukaryotic-based synergistic formulation for gastro-intestinal disorders.

The other object of the present invention is to provide a method for producing a eukaryotic-based formulation that eliminates the shortcomings of the existing formulations that are generally base on prokaryotics.

Other object of this invention is to provide a method for producing a formulation that regulates the balance of microbial flora in the gastrointestinal tract of mammals.

Another object of the present invention provides a method for producing a eukaryotic-based probiotic formulation with more stability and efficacy.

Still other object of the present invention is to provide a method for producing a formulation, which reduces if not prevents the ill effects of the harmful organisms.

Yet other object of the present invention is to provide a method for producing a formulation that stimulates the growth of probiotics and inhibits the growth of undesired harmful microbes.

Yet another object of the present invention is to provide a method for producing a single formulation, which can take care of secondary ill effects induced through ingestion or administration of pharmaceuticals or physiological components, to which the gut-micro-flora is associated.

Still another object of the present invention is to provide a method for producing a formulation where in an introduction of protective barrier is eliminated.

Yet another object is to provide a method for producing a formulation that is user friendly and has improved compliance by combining two drugs in one formulation. Further the drugs are complementary to each other. Since the components are incorporated in single formulation, they get administered together and thus the action gets synchronized.

Further object is to provide a method for producing a formulation that releases the components at desired site.

In another aspect of this invention there is provided a process for isolating, modifying, culturing, lyophilizing, drying and preparing formulations using eukaryotic organisms.

Accordingly the present invention provides a method for producing a eukaryotic based synergistic formulation for gastro-intestinal disorders comprising eukaryotics and a further pharmaceutically or physiologically acceptable component, wherein the eukaryotic used is *Saccharomyces boulardii (S. boulardii),* said method comprising the steps of:
- isolating *S. boulardii* from Ananas comosus (pineapple)
- cultivating the isolated *S. boulardii* in a medium designated as BBIL-SB comprising glucose for carbon source, soybean casein dextrose medium (SCDM) for nitrogen source, MgSO₄, KCl, NaCl, (NH)₄HPO₄ and with microelements ranging from 0.001% to 0.6% and being selected from MnSO₄, FeSO₄, CuSO₄, boric acid, Vitamins, D-Biotin and thiamine HCl and
- lyopohilization drying and granulation of the cultivated *S. boulardii,* wherein no encapsulation of the eukaryotic is required.

Said *S. boulardii* may be native or further modified.

In one embodiment physiologically acceptable components may include without restricting to physiologically acceptable any known excipients exemplified by magnesium stearate; growth stimulators, stabilizers such as lactose; nutritional supplements such as vitamins, minerals, chitosans and source of lactic acid producers.

In yet another embodiment pharmaceutically acceptable components may be such as anti-infective agents that can be used for therapeutic purpose and include antibiotics like Ampicillin, Amoxycillin, Cloxacillin, Clavulanic acid, Cephalexin, Cefuroxime, Axetil, and Cefixime; antiviral agents exemplified by Acyclovir, Zidovudin and Lamivudin.

In a further embodiment the invention advises of incorporating a source of lactic acid producers such as lactobacillus in lyophilized form or curd or buttermilk.

The formulation may contain eukaryotics in the range of 0.1 to 5 billions cells. The lactic acid producers may be employed in the range of 0.1 to 1.0 billion cells and the pharmaceutically acceptable components may be added in the quantity required for therapeutic purposes, generally, antibiotics may be added up to 500 mg.

The other physiologically acceptable components may be added as per requirement.

The formulation can be converted to capsules, tablets or powder. The tablets may be coated with conventional excipients. The organisms may also be coated separately to isolate the same from other ingredients to enhance its potency.

***Isolation of S.boulardii:*** The isolation media consists of yeast extract, peptone, Glucose in concentration ranging from 0.5 to 2%. Pieces of fruits were transferred to the medium and incubated from 24-72 hrs at 22-25 °C. The mixed culture was streaked on soybean digest agar (SDA) and soybean casein digest agar (SCDA) petriplates and incubated for 24-72hrs at 22-25 °C. Morphologically identified yeast colonies were again streaked in SDA medium with 1-1.5% tetrazolium salt and incubated for 24-72 hrs at 22-25 °C.

The entire process of isolation of the eukaryotic organism is done by any conventional process known to those skilled in the art.

***Primary Culture of S.Boulardii:*** Pinkish colonies were isolated and transferred in 10 ml yeast extract, peptone and Glucose in concentration ranging from 0.5% to 2%, and incubated at 20- 25 °C for 24-36 hrs at 150-200 rpm.

***Secondary Culture of S.Boulardii:*** 3-5% inoculum was transferred from the primary culture and transferred to 90-100 ml YPD medium and incubated at earlier conditions.

***Storage of the Culture:*** Subsequent to centrifugation, the pellet is resuspended in 50% glycerol in Water For Injection (WFI), aliquoted and stored in cryovials at -85 °C.

***Large scale culture of Boulardii:*** A single cryovial was inoculated in 50 ml of **Bharat Biotech Int. Ltd-** ***S.Boulardii*** (BBIL-SB) defined media consisting of Glucose for carbon source, soybean casein dextrose medium (SCDM) for Nitrogen source, MgSO₄, KCI, NaCl, (NH)₄HPO₄ and with microelements like MnSO₄, FeSO₄, CuSO₄, Boric acid and Vitamins, D-Biotin and thiamine HCl ranging from 0.001% to 0.6% and incubated at 25-30 °C, 200-250 rpm and incubated for 24-36 hrs. After said period the culture was used as inoculum for cultivating 10 liters of the same BBIL-SB medium in fermentor.

The parameters maintained during fermentation, Temp. 30+/-2 °C, pH 5.2+/- 0.2, air flow 0.5 to 1.5 VVM and agitation ranging from 300-700 rpm. Further Fed batch growth was carried in the same BBIL-SB medium with 70% glucose. After 40 hrs of cultivation the culture was harvested and centrifuged, the pellet was washed with normal saline and resupended in normal saline.

The entire process of primary culture, secondary culture, storage and fermentation are done by process known to those skilled in the art.

**Lyophilization:** The microorganisms were suspended in various stabilizing agents at final concentration to maintain the viability and stability. These were granulated under vacuum by parameters known to those skilled in the art. The dried powder was checked for the moisture content and viability.

**Fluidized bed drying:** The cells were mixed with stabilizing agents and screened for the required granules sizes and dried for the required time.

The above said detailed process of isolation, primary culture, secondary culture, storage, preservation of viable cells can be applied for the yeast or other eukaryotic organism which have its application in curative therapy for gastro-intestinal disorder.

However it is not intended that the scope of the invention be limited by the said examples. Any amendments obvious to a person skilled in the art may be effected to get the proper strain of the yeast.

**Formulation:** Various formulations were prepared as detailed below in the examples involving in house produced yeast *(S. boulardii)* or the yeast procured from commercial sourcesin dry powdered form with granular size in the range of 14-30 ASTM. The drying may be carried out either by lyophilisation, fluid bed drying, vacuum drying or spray drying.

The formulation was shaped to Capsules, Tablets, Dispersible tablet formulation, Dry powder formulations.

**I) Capsules:** dehydrated eukaryotics and other adjuncts as specified in various examples are lumped together and then encapsulated by any known methods.

**II) Tablets:**
a) ***Coated Tablets:** S.Boulardii* or other Eukaryotic organism together with other ingredients such as acceptable excipients are lumped and compressed in to tablets. The tablets thus produced are further coated. During the coating stage the pharmaceutically acceptable components such as anti-infective agents are introduced. It is also be done vice versa.
b) ***Layered tablets:** S. Boulardii* or other Eukaryotic organism organisms are compressed with auxiliaries including excipients and optionally coated into one layer of tablet. The other layer(s) of tablet contains the pharmaceutically acceptable components such as anti-infective agent. The second layer is placed above the first layer.
c) ***Tablet containing mixture*:** Granules of *S.boulardii* or eukaryotic organism barrier film and mixed with granulated material of anti-infective agents and compressed into a tablet.
d) ***Tablet with a hole:*** The infective agent is made into a tablet with a Hole. The hole of the tablet is filled with organisms. The tablet so obtained may be coated for final finishing.
e) ***The tablet is coated with a barrier film*:** The film protected organisms are introduced into the capsule independently. i.e. anti-infective agent is included in coating.

**III) Dispersion tablet formulation:** A liquid tablet is prepared using the following components of which the active ingredients are anti-infective agent(s) and micro-organisms. One of the active ingredients is granulated after suspending. The eukaryotic organisms or anti-infective agent are coated with barrier film and if necessary may be mixed with other ingredients of formulation. The barrier film is stable in liquid formulation but disintegrates in body due to physiological conditions. These agents are: Cellulose acetate phthalate, Hydrogenated Castor Oil, Cetyl Alcohol, Diethyl Phthalate, Ethyl cellulose, Hydroxypropyl Cellulose, Hydroxypropyl Methylcellulose Phthalate and Zein. It is not intended **that the scope of this invention be limited by these examples agents only.**

The product is reconstituted before use by addition of adequate amount of liquid.

**IV) Dry powder formulations:** A dry powder for reconstituting liquid formulation before use is prepared using the following components of which the active ingredients are anti-infective agent(s) and micro organisms. The remainning components are physiologically acceptable excipients

### ORAL DOSE FORMULATION I:

**Capsules:** Stable dose combination capsules are prepared using following components of which the active ingredients are eukaryotic organism like yeast and other anti-infective agents. The remaining components are physiologically acceptable excipients.
The capsules were stored at room temperature (RT) and 40°C. The selection of the temperatures is based on the International Conference on Harmonization (ICH guidelines).

The viability of 40°C.+/-2 (75% RH+/-5+/-) was estimated in the different formulation during the entire time period. The stability of the product at these temperatures can be extrapolated to the shelf life of 2yrs.

The viability of the microorganism was tested by the serial dilution and pour plate methods. These methods can be done by those skilled in the art.

Antibiotics were assayed by the methods as described in USP27.

Following are examples illustrate the ingredients and their proportions for preparing various formulations. All formulations contain eukaryotics in the range of 0.1 to 5 billion cells. However, it is not intended that the scope of this invention be limited by these examples.

### Example 1a:

The formulation had 1-20 mg of lactose and 0.5 -2.85 mg of magnesium stearate.

### Example 1 b:

The formulation had 1-20 mg lactose, 0.5 -2.85 mg Magnesium stearate and each 10-50 mcg of vitamins A,K,C and minerals like 1 mcg-10 mg of divalent and trivalent metals like Zinc, Iron, and Copper and 5- 20 mg of Chitosan.

### Example 1c:

The formulation had 20 mg lactose, 2.85 mg Magnesium stearate and each 10-50 mcg of vitamins A, K, C and minerals like 1 mcg -10 mg of divalent and trivalent metals like Zinc, Iron, and Copper.

### Example 1d:

The formulation had 1-20 mg lactose, 0.5-2.85 mg Magnesium stearate and each 10-50 mcg of vitamins A, K, C.

### Example 1e:

The formulation had 1-20 mg lactose, 0.5-2.85 mg Magnesium stearate and minerals like 1mcg-10 mg of divalent and trivalent metals like Zinc, Iron, and Copper.

### Example 1f:

The formulation has 1-20 mg Lactose, 0.5-2.85 mg Magnesium stearate and 5-20 mg of Chitosan.

### Example 1g:

The formulation had 1-20 mg lactose, 0.5- 2.85 mg Magnesium stearate and minerals like 1 mcg-10 mg of divalent and trivalent metals like Zinc, Iron, and Copper and 5-20 mg Chitosan.

### Example 1h:

The formulation had 1-20 mg lactose, 0.5-2.85 mg Magnesium stearate and each 10-50 mcg of vitamins A, K, C and antibiotics like 100-250 mg Amoxycillin and 100-250 mg Cephalexin.

### Example 1i:

The formulation had 1-20 mg lactose, 0.5-2.85 mg Magnesium stearate and minerals like 1 mcg -10 mg of divalent and trivalent metals like Zinc, Iron, and Copper and antibiotics like 100-250 mg Amoxycillin and 100-250 mg Cephalexin, 5-20 mg Chitosan and 10-50 mcg of vitamins A, K, C.

### Example 1j:

The formulation had only 0.1-5 billions *S.Boulardii* cells required as curative dose without any excipients or Stabilizers or another agent which can contribute to stability to *S.Boulardii.*

### Example 1k:

The formulation has antibiotics like 0-500 mg Amoxycillin and 0-500 mg Cephalexin only.

### Example 1I:

The formulation had 0.1- 1 billion cells of *Lactobacillus* only with other fillers such as starch wherein the amount of starch varies depending on the size of the capsule.

### Example 1m:

The formulation had 0.1-1.0 billion cells of *Lactobacillus* and 0-500 mg anti-infective agents like either amoxycillin or Cephalexin.

### Example 1n:

The formulation had encapsulated 0.1-1.0 billion cells of *Lactobacillus* and encapsulated 0-500 mg of either Amoxycillin or 0-500 mg Cephalexin anti-infective agents.

### Example 1o:

The formulation had 0.1 - 5 billions cells of *S.Boulardii, Lactobacillus* and anti-infective agents either 0-500 mg Amoxycillin or 0-500 mg Cephalexin.

### Example 1p:

The formulation had only 0.1-5 billion cells of *S.Boulardii* and 0.1-1.0 billion cells of *Lactobacillus* only.

### Example 1q:

The formulation had 0.1-5 billion cells of *S.Boulardii* and 100-800 mg Acyclovir.

### Example 1r:

The formulation had 0.1-5 billion cells *S.Boulardii* and 50-400 mg Zidovudine.

### Example 1s:

The formulation had 0.1-5 billion cells *S.Boulardii,* 50-250 mg Lamivudine.

Results: The viability and the potency of the infective agent tested in each formulation at the beginning of the stability study, in between and at the end of the stability study was conducted. Results confirm the presence of viability and potency values comparable to the challenged limits and the values showed no significant decrease in examples 1 a to 1k, wherein all the formulations had only *S.Boulardii or S.boulardii* with other agents such as either anti-infective agents, Vitamins or stabilizers.

In case of example 1 m the viability of the *Lactobacillus* did not decreased significantly confirming either the ability of the micro organism to withstand the accelerated temperature and no action of the anti-infective agent in the formulation. There was no significant drop in the potency of the anti-infective agent.

The stability of the formulation of example 1 n, also showed significant decrease in the activity confirming the inability of the barrier to prevent the action of the anti-infective agent on the bacteria or the in stability of the bacteria to withstand elevated temperatures.

Similarly no significant drop of viability and maintenance of potency was observed in the example 1o and 1p, Observation revealed the maintenance of the viability of the yeast and bacteria during the entire period of the experimentation, confirming no action of the infective agent on the bacteria nor yeast.

Finally results of formulation of probiotic yeast with anti-viral agent as cited in examples 1q, 1r, 1s clearly showed no significant drop in activity over the entire period of experimentation.

### ORAL DOSAGE FORMULATION II:

**Tablet:** Stable fixed dose combination layered tablet is prepared using the following components of which the ingredients are anti-infective agent(s) and yeast or other eukaryotic organisms. If necessary, acceptable excipients can be added.

One of the active ingredients is coated. The entire process of tablet making including the selection of the temperature, humidity and other stability deciding parameter are done by process known to those skilled in the art.

Following are examples of providing barrier to organisms for different dosage forms. However, it is not intended that the scope of this invention be limited by these examples.

### Example 2a:

The formulation had encapsulated antibiotics like 0-500 mg Amoxycillin, 0-500 mg Cephalexin with granulated yeast of 0.1 to 5 billion.

### Example 2b:

The formulation had antibiotics like 0-500 mg Amoxycillin, 0-500 mg of Cephalexin and encapsulated *S. boulardii* of 0.1 to 5 billion cells.

### Example 2c:

The formulation had encapsulated antibiotics like 0-500 mg Amoxycillin or 0-500 mg Cephalexin along with encapsulated *S. boulardii* 0.1-5 billion cells of yeast.

### Example 2d:

The formulation had 0.1-5 billion cells of *S.Boulardii* antibiotics and 0-500 mg and or 0-500 mg of anti-infective agents.

### Example 2e:

The formulation had 0.1-5 billion cells of *S. Boulardii* with 50-800 mgs of either Acyclovir, Zidovudine and Lamivudine.

**Results:** Data confirms the maintenance of viability of the organism and also the retainment of the potency of the anti-infective agents.

This clearly indicated the stability and its ability of the formulation to dissolve at the intestinal physiological pH, confirming the usage of this formulation as curative for gastrointestinal disorders.

**Results:** Data confirms the maintenance of viability of the organism and also the retainment of the potency of the anti-infective agents.

This clearly indicated the stability and its ability of the formulation to dissolve at the intestinal physiological pH, confirming the usage of this formulation as curative for gastrointestinal disorders and also as curative therapy for viral infections by inhibiting DNA synthesis, viral replication and inhibition of HIV reverse transcription via viral DNA chain termination.

### DOSAGE FORMULATION III:

**Dispersion tablet formulation:** Stable fixed dose combination liquid tablet is prepared using the components of which the active ingredients are anti-infective agent(s) and micro organisms.

The anti-infective agent is granulated, and the other ingredient is introduced into the formulation by the process known to those skilled in the art.

This dose suspension provides pharmacological effects when kept at conditions suggested by ICH guidelines which could be extrapolated to the self life at ambient room temperature.

Following are examples of providing barrier to organisms for different dosage forms. However, it is not intended that the scope of this invention be limited by these examples.

**Example 3a:** The relative proportion of active ingredients either anti-infective agent (either 0-500 mg Ampicillin or 0-500 mg Cephalexin) and eukaryotic microorganisms along with other stabilizers. Additives or other pharmacopial or Non-pharmacopial agents (Cellulose acetate pthalate, Isopropyl alcohol and Dichloromethane) which can impart the stability, maintenance, increase or decrease the viability of the organism.

**Results:** Data from the study clearly indicate no significant loss of the viability of the microorganism or the potency of the anti-infective agent during the entire period of accelerated stability study suggesting the beneficial effect of the formulation during storage at ambient temperature through the shelf life.

### DOSAGE FORMULATION IV:

### Stability of the formulation in liquid after reconstitution

Following are examples of providing barrier to organisms for different dosage forms. However, it is not intended that the scope of this invention be limited by these examples.

**Example 4a:** Stable fixed dose combination dry powder for reconstituting liquid formulation before use is prepared using the following components of which the active ingredients are anti-infective agent(s) and micro organisms. The remaining components are physiologically acceptable excipients.

In present invention, Physiologically accepted quantities of *S.Boulardii* are granulated by process known to those skilled in the art. The granules are dried and mixed with dry powder containing 0-500 mg Amoxicillin or and 0-500 mg Cephalexin with or without other agents like 0-500 mg Hydroxypropyl methyl cellulose by processes known to those skilled in the art.

The powder at different time intervals were subjected to cell viability and antibiotic assay by both microbiological methods and Instrumentation methods. These methods are known to those skilled in the art.

Stability of the dry powder at the accelerated temperatures was carried out as prescribed by ICH guidelines.

**Results:** Data from the study clearly indicate the retainment of the viability of the organism and also the content of the antibacterial agent in the powder.

This clearly indicates the conferment of powder stability indicating the usage of the presentation as a curative therapy for gastrointestinal disorders.

### ADVANTAGES:

❖ The formulation is stable, user-friendly & safe.
❖ No encapsulation is required. The components can be mixed and not required to be isolated from one another. It becomes effective at a desired site.
❖ The formulation is effective & efficient against harmful microflora inhabitant to gastrointestinal tract and their metabolites and secretions.
❖ The formulation is supportive to probiotics inhabitant to gastrointestinal tract.

## Claims

1. A method for producing a eukaryotic based synergistic formulation for gastro-intestinal disorders comprising eukaryotics and a further pharmaceutically or physiologically acceptable component, wherein the eukaryotic used is *Saccharomyces boulardii (S. boulardii),* said method comprising the steps of:
- isolating *S. boulardii* from *Ananas comosus* (pine apple),
- cultivating the isolated *S. boulardii* in a medium designated as BBIL-SB comprising glucose for carbon source, soybean casein dextrose medium (SCDM) for nitrogen source, MgSO₄, KCI, NaCl, (NH)₄HPO₄ and microelements ranging from 0.001% to 0.6% and being selected from MnSO₄, FeSO₄, CuSO₄, boric acid, vitamins, D-biotin and thiamine HCl, and
- lyophilization drying and granulation of the cultivated *S. boulardii,* wherein no encapsulation of the eukaryotic is required.

2. The method according to claim 1, wherein *S. boulardii* used is native or further modified.

3. The method according to any of claims 1 to 2, wherein the physiologically acceptable components include magnesium stearate, growth stimulators, stabilizers, nutritional supplements, vitamins, minerals, chitosans and source of lactic acid producers.

4. The method according to any of claims 1 to 3, wherein pharmaceutically acceptable components are anti-infective agents comprising antibiotics, preferably Ampicillin, Amoxycillin, Cloxacillin, Clavulanic acid, Cephalexin, Cefuroxime, Axetil, and Cefixime, and antiviral agents selected from Acyclovir, Zidovudin and Lamivudin.

5. The method according to claim 3, wherein the lactic acid producers are in lyophilized form or its source such as curd, or buttermilk.

6. The method according to any of the preceding claims, wherein the eukaryotics are present in the range of 0.1 to 5 billion cells.

7. The method according to claim 5, wherein the lactic acid producers are present in the range of 0.1 to 1.0 billion cells.

8. The method according to any of the preceding claims, wherein the pharmaceutically acceptable components are present in the quantity required for therapeutic purposes, wherein the pharmaceutically acceptable components are antibiotics.

9. The method according to claim 8, wherein antibiotics are present up to 500 mg.

## Patentansprüche

1. Verfahren zur Herstellung einer synergistischen Formulierung auf eukaryotischer Basis gegen gastrointestinale Erkrankungen, beinhaltend Eukaryoten und eine weitere pharmazeutische oder physiologisch geeignete Komponente, wobei der verwendete Eukaryot *Saccharomyces boulardii (S. boulardii)* ist, und das Verfahren folgende Schritte umfasst:
- Isolierung von *S. boulardii* aus *Ananas comosus* (Ananas),
- Kultivierung der isolierten *S. boulardii* in einem Medium bezeichnet als BBIL-SB, beinhaltend Glucose als Kohlenstoffquelle, Sojabohnen Casein Dextrose Medium (SCDM) als Stickstoffquelle, MgSO₄, KCl, NaCl, (NH)₄HPO₄ und Spurenelemente in einer Konzentration von 0.001% bis 0.6%, ausgewählt aus MnSO₄, FeSO₄, CuSO₄, Borsäure, Vitamine, D-Biotin und Thiamin HCl, und
- Lyophilisation und Granulierung der kultivierten *S. boulardii,* wobei keine Verkapselung des Eukaryoten notwendig ist.

2. Verfahren nach Anspruch 1, **dadurch gekennzeichnet, dass** *S. boulardii* natürlich oder weiter modifiziert eingesetzt wird.

3. Verfahren nach Anspruch 1 oder 2, **dadurch gekennzeichnet, dass** die physiologisch geeigneten Komponenten Magnesium Stearate, Wachstumsstimulatoren, Stabilisatoren, Nahrungsergänzungsmittel, Vitamine, Mineralien, Chitosane und eine Quelle von Milchsäureproduzenten umfassen.

4. Verfahren nach einem der Ansprüche 1 bis 3, **dadurch gekennzeichnet, dass** die pharmazeutisch geeigneten Komponenten anti-infektiöse Agenzien sind, umfassend Antibiotika, bevorzugt Ampicillin, Amoxycillin, Cloxacillin, Clavulansäure, Cephalexin, Cefuroxime, Axetil und Cefixime, sowie antivirale Agenzien wie Acyclovir, Zidovudin und Lamivudin.

5. Verfahren nach Anspruch 3, **dadurch gekennzeichnet, dass** die Milchsäureproduzenten in lyophilisierter Form oder in Form ihrer Quelle wie Quark oder Buttermilch vorliegen.

6. Verfahren nach einem der vorherigen Ansprüche, **dadurch gekennzeichnet, dass** die Eukaryoten in einem Bereich von 0.1 bis 5 Milliarden Zellen vorhanden sind.

7. Verfahren nach Anspruch 5, **dadurch gekennzeichnet, dass** die Milchsäureproduzenten in einem Bereich von 0.1 bis 1 Milliarden Zellen vorhanden sind.

8. Verfahren nach einem der vorherigen Ansprüche, **dadurch gekennzeichnet, dass** die pharmazeutisch geeigneten Komponenten in einer Quantität vorhanden sind, die für therapeutische Zwecke benötigt wird, wobei die pharmazeutisch geeigneten Komponenten Antibiotika sind.

9. Verfahren nach Anspruch 8, **dadurch gekennzeichnet, dass** Antibiotika in einer Konzentration bis zu 500 mg vorhanden sind.

## Revendications

1. Procédé de production d'une formulation synergique à base d'eucaryotes destinée aux troubles gastro-intestinaux qui comprend des eucaryotes et un autre composant pharmaceutiquement ou physiologiquement acceptable, dans lequel l'eucaryote utilisé est *Saccharomyces boulardii (S. boulardii),* ledit procédé comprenant les étapes consistant à :
- isoler *S. boulardii d'Ananas comosus* (ananas),
- cultiver *S. boulardii* isolé dans un milieu, dénommé BBIL-SB, comprenant du glucose en tant que source de carbone, un milieu soja-caséine-dextrose (SCDM) en tant que source d'azote, du MgSO₄, du KCl, du NaCl, du (NH)₄HPO₄ et des microéléments dans la plage de 0,001 % à 0,6 % et choisis parmi le MnSO₄, le FeSO₄, le CuSO₄, l'acide borique, les vitamines, la D-biotine et la thiamine HCl, et
- lyophiliser et granuler les *S. boulardii* issus de la culture,
aucune encapsulation de l'eucaryote n'étant nécessaire.

2. Procédé selon la revendication 1, dans lequel *S. boulardii* utilisé est natif ou modifié.

3. Procédé selon l'une quelconque des revendications 1 à 2, dans lequel les composants physiologiquement acceptables comprennent le stéarate de magnésium, des stimulateurs de croissance, des stabilisants, des suppléments nutritionnels, des vitamines, des minéraux, des chitosanes et une source de producteurs d'acide lactique.

4. Procédé selon l'une quelconque des revendications 1 à 3, dans lequel les composants pharmaceutiquement acceptables sont des agents anti-infectieux comprenant des antibiotiques, de préférence l'ampicilline, l'amoxicilline, la cloxacilline, l'acide clavulanique, la céphalexine, le céfuroxime, l'axétil, et le céfixime, et des agents antiviraux choisis parmi l'acyclovir, la zidovudine et la lamivudine.

5. Procédé selon la revendication 3, dans lequel les producteurs d'acide lactique sont sous une forme lyophilisée ou sa source comme un précipité caillebotté, le babeurre.

6. Procédé selon l'une quelconque des revendications précédentes, dans lequel les eucaryotes sont présents dans la plage de 0,1 à 5 milliards de cellules.

7. Procédé selon la revendication 5, dans lequel les producteurs d'acide lactique sont présents dans la plage de 0,1 à 1,0 milliard de cellules.

8. Procédé selon l'une quelconque des revendications précédentes, dans lequel les composants pharmaceutiquement acceptables sont présents en la quantité requise pour des fins thérapeutiques, les composants pharmaceutiquement acceptables étant des antibiotiques.

9. Procédé selon la revendication 8, dans lequel les antibiotiques sont présents en une quantité pouvant atteindre 500 mg.
